# EUROPEAN PATENT SPECIFICATION

(11) **EP 3 873 508 B1**
(45) Date of publication and mention of the grant of the patent: **18.11.2026**
(21) Application number: 19880371.0
(22) Date of filing: 09.01.2019
(51) Int. Cl.: A61K 36/38, A61K 36/30, A61K 36/15, A61K 35/644, A61K 33/06, A61K 36/22, A61K 35/12, A61K 9/06, A61P 17/06, A61K 35/35, A61K 47/44, A61K 9/00

(54) **A MEDICINE FOR TREATMENT OF PSORIASIS AND PRODUCTION METHOD THEREOF**
ARZNEIMITTEL ZUR BEHANDLUNG VON PSORIASIS UND VERFAHREN ZU DESSEN HERSTELLUNG
MÉDICAMENT POUR LE TRAITEMENT DU PSORIASIS ET SON PROCÉDÉ DE PRODUCTION

(30) Priority: 01.11.2018 TR 201816367
(43) Date of publication of application: 08.09.2021
(73) Proprietor: Yeditepe Universitesi, 34755 Istanbul (TR)
(72) Inventor: SAHIN, Fikrettin, Istanbul (TR); KASAPOGLU, Burcu, Istanbul (TR); TÜRKEL SESLI , Nezaket, Istanbul (TR); SAHBAZ, Çigdem Dilek, Istanbul (TR); TURGUT, Cengiz, Bayburt (TR)
(74) Representative: Sevinç, Cenk
(86) International application number: PCT/TR2019/050019
(87) International publication number: WO 2020/091703

(56) References cited:
- WO-A1-2014/071426
- WO-A2-2010/100651
- CN-A- 1 543 995
- CN-A- 104 623 307
- DE-U1- 202005 009 813
- US-A- 4 325 965
- BARNES, T. M. ET AL.: "Topical pine tar: History, properties and use as a treatment for common skin conditions", AUSTRALASIAN JOURNAL OF DERMATOLOGY, vol. 58, no. 2, 2017, pages 80 - 85, XP55704455

## Description

### Field of the Invention

The present invention relates to a medicine for use in the treatment of psoriasis and production method thereof.

### Background of the Invention

Psoriasis is a systemic inflammatory disease characterized by white colored skin eruption on a red colored base. Psoriasis is mostly seen at the knees and elbows and causes severe itching in the patients. Although it is not clear what causes psoriasis which is a non-infectious disease, it is known that the stress factor has a triggering effect on psoriasis in cases where the body's defense mechanism fails.

In 2008, observing that a cream containing Imiquimib (IMQ) active ingredient (Aldara contains 5% Imiquimib, commercial name) used topically to treat genital warts has the side effect of inducing psoriasis-like lesions in humans, and noticing the same effect on mice have attracted attention to this cream **[1].** It has been well demonstrated by the studies that IMQ, which binds to Toll-like TLR7 and TLR8 receptors and activates the systemic inflammation pathway as an immune activator, produces a systemic response after topical use in mice leading to formation of psoriasis **[2, 3, 4, 5, 6].** Nowadays, it has been decided to show the six main points in mice model histopathologically and immunohistochemically for the efficiency and standardization of the preclinical studies for psoriasis. These are as follows:
1. Observing the change in epidermal keratinocyte hyperproliferation and epidermal differentiation,
2. Papillomatosis,
3. Infiltration of T-cells, dendritic cells, macrophages and neutrophils,
4. Observing functional roles of the T-cells,
5. Changes in the dermal vascularization and
6. Ability to exhibit response to the agents against the disease.

Currently, the IMQ-induced mouse model is used as an ideal preclinical model that follows the said six points.

Although there is no treatment that completely cures psoriasis yet, it is tried to be controlled by the dermatologists with various drugs. The subcutaneous immunological agents, which have been started to be used recently, have unfortunately not been a common and favorable option in the treatment of the disease due to their serious side effects (such as immunosuppression, toxicity, etc.), hospitalization and follow-up difficulties, serious costs and inability to prevent recurrence of the disease **[7].**

CN 1 543 995 A discloses an ointment for the treatment of psoriasis comprising a mixture of plant-based active components including Alkanna tinctoria.

### Summary of the Invention

The objective of the present invention is to develop a drug formulation for treatment of psoriasis. This objective is achieved by the subject-matter of independent claims 1 and 4.

### Detailed Description of the Invention

"A medicine for treatment of psoriasis and production method thereof" developed to fulfill the objective of the present invention is illustrated in the accompanying figures, in which;
- **Figure 1.**: is a graphical representation of epidermis thickness.
- **Figure 2.**: is a graphical representation of the analysis of lymphocyte proliferation.
- **Figure 3.**: is a graphical representation of the T regulator cell analysis in lymphocyte culture.
- **Figure 4.**: is a graphical representation of the spleen versus body weight ratios.
- **Figure 5.**: is a graphical representation of the change in the ear thickness throughout the experiment.
- **Figure 6.**: is a graphical representation of the change in the redness on the skin throughout the experiment.
- **Figure 7.**: is a graphical representation of the change in the thickness on the skin throughout the experiment.
- **Figure 8.**: is a graphical representation of the change in the plaque formation on the skin throughout the experiment.

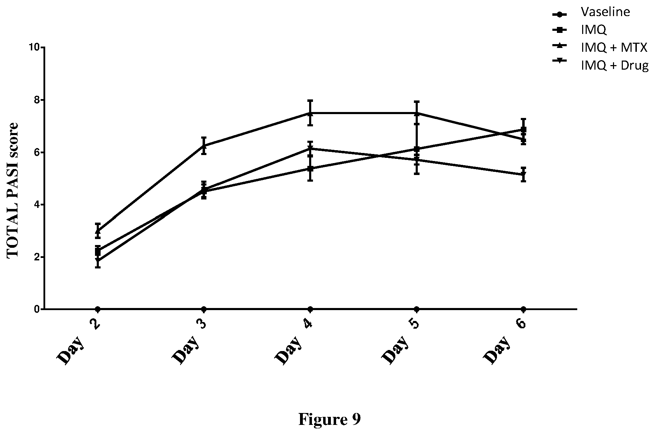

- **Figure 9.**: is a graphical representation of the change in the total Psoriasis Area Severity Index (PASI) throughout the experiment.

The psoriasis medicine of the present invention preferably comprises 800 to 1000 g of tallow, 250 to 350 g of larch resin, 450 to 550 g of beeswax, 2 to 3 g of gum mastic, 40 to 50 g of propolis, 200 to 250 g of Alkanna tinctoria, 200 to 250 g of alum, 150 to 200 g of Juniper tar. The production method of the psoriasis medicine of the present invention comprises the steps of
- Melting the tallow at 300°C by continuously mixing it with the beeswax,
- Adding first the gum mastica and then the propolis to the obtained molten mixture,
- Adding ground larch resin to the mixture together with alum,
- Finally adding ground Alkanna tinctoria and juniper tar to the mixture,
- Obtaining a homogenous mixture by means of continuous mixing,
- Filtering the homogenous mixture,
- Obtaining the medicine for treatment of psoriasis, which is the final product in the form an elute.

In the preferred embodiment of the invention, the obtained elute is applied as an ointment by spreading it on the surface of the skin of the psoriasis patient where the lesion is observed.

### Experimental Studies

### Modeling Psoriasis in Mouse and Application of the Drug

The cream named Aldara (Meda Pharma, 3M Health Care) containing 5% Imiquimib (IMQ) was administered once a day for 6 days on the shaved backs and right ears of 8-11 weeks old adult male BALB/c mice such that a total of 62.5 mg cream is applied per day per animal thereby psoriasis inflammation was produced in the animal model. Vaseline was applied to the shaved area of the animals in the control group. The positive control group received 1 mg/kg Mtx (Methotrexate) orally per day. The drug was administered orally at a dose of 5 mg/kg once a day during the experiment. Administration of the drug to the animals in the group where the semi-therapeutic effect of the drug was to be tested was initiated 1 day after the onset of induction of psoriasis with IMQ, and continued until the end of the experiment period. Body weight, and redness, flaking and thickening on the skin were daily measured and scored according to the Psoriasis Area Severity Index (PASI) to monitor the general health status of the animals and the induction of psoriasis with IMQ. As required by the model, thickening of the right ear skin was also recorded on a regular basis since the day the experiments began.

At the end of the application, animals were anesthetized by isoflurane and then euthanized by cervical dislocation. The size and weight of the spleen tissues were recorded.

### Epidermis Thickness Analysis

The skin samples collected from the IMQ-treated backs of the mice after dissection were fixed in formalin, and paraffin blocks were prepared. Ten sections having a thickness of 10 microns were collected at equal intervals from each animal. The sections were stained with Masson Trichrome and their images were taken by microscope. The epidermis thicknesses were calculated by taking the average of the measurements taken at 5 different points for each section.

### T Lymphocyte Isolation, Staining and Stimulation from the Spleen Tissue

After dissection, the spleen tissues were divided into smaller parts by a scalpel in a petri dish containing RPMI medium, then they were thoroughly ground with a pasteur pipette and washed without being allowed to disintegrate. After the cell suspension was passed through a 70 µm filter, it was taken up in PBS and centrifuged for 10 minutes at 2000 rpm. The precipitated cells were dissolved in sterile lysis solution containing 0.037 g/L EDTA, 1 g/L potassium bicarbonate and 8.29 g/L ammonium chloride, and incubated at room temperature for 10 minutes. The supernatant obtained after centrifugation at 800 rpm for 10 minutes was dissolved in PBS and centrifuged at 2000 rpm for 10 minutes. The precipitated cells were dissolved in 10 ml of cRPMI medium and the number of cells was determined.

CFSE was placed on the isolated cells for staining purposes. This process and the rest were carried out in dark environment. Cells incubated at +4°C for 6 minutes were then placed in cRPMI and centrifuged at 2000 rpm for 5 minutes. cRPMI was added to the precipitated cells and they were again centrifuged at 1200 rpm for 5 minutes. The precipitated cells were resuspended in cRPMI medium and the number of cells was determined. 55 x 10⁵ cells were seeded in each well. The lymphocytes were stimulated with CD3 and CD28. The cultures were placed in a 37°C incubator for 3 days.

### Proliferation Analysis of Lymphocytes

The isolated lymphocytes were spread into each well of 48-well plates at a concentration of 5 x 10⁵ cells per well. Lymphocytes stained with CFSE were cultured for 3 days for proliferation analysis. Non-stimulatory and anti-CD3+CD28 (CDmix) stimulated cultures of lymphocyte cells in each group were performed. On the third day, proliferation analyses of the lymphocyte cells were examined by flow cytometry. CFSE fluorescence staining can be displayed in FL-1 in flow cytometry. After the analyses, proliferation of the T cells was compared in the presence or absence of stimuli.

### T Regulator Cell Analysis in Lymphocyte Cultures

After 3 days of culturing of the isolated lymphocyte cells, CD4⁺CD25⁺FoxP3⁺ cell numbers were examined. After enabling the cells to be homogenously dissociated by pipetting in the wells, they were taken out of this homogenous solution and put into flow tubes. The cells, to which PBS was added, were centrifuged at 1200 rpm for 5 minutes. CD4 and CD25 were added to the tubes and vortexed and were incubated at room temperature in the dark for 20 minutes. Then the staining buffer solution was added and centrifuged at 250 g for 10 minutes. FoxP3 buffer solution was added to the precipitated cells and they were vortexed and incubated at room temperature in the dark for 10 minutes. They were then centrifuged at 500 g for 5 minutes. Staining buffer solution was added to the precipitated cells and they were vortexed and centrifuged at 500 g for 5 minutes. FoxP3 buffer solution C was added to the precipitated cells and they were vortexed and incubated at room temperature in the dark for 30 minutes. Then the staining buffer solution was added and centrifuged at 500 g for 5 minutes. This process was repeated once more for the precipitated cells. Then the FoxP3 antibody was added and the vortex process was carried out slowly. They were incubated at room temperature in dark for 30 minutes and then the staining buffer solution was added and centrifuged at 500 g for 5 minutes. The staining buffer solution was added to the precipitated cells and they were analyzed in the flow cytometry device.

In the literature, the use of drugs prepared with traditional methods in psoriasis is frequently seen **[3].** Especially in Chinese and Indian medicine, certain herbal mixtures have been used in this field for centuries. Recently, it has been observed that these traditional drugs are being tested in controlled preclinical studies. An important reason for this is the ease of use of the standardized IMQ-mouse model which has become widely preferred for evidence-based medicine practices in psoriasis. When we study the chemical behavior of these herbal compounds that are traditionally used in psoriasis and similar skin diseases, the strong anti-inflammatory properties of these mixtures stand out. These anti-inflammatory systemic features exhibit promising although limited number of positive data in both preclinical and clinical histopathology **[3].** We demonstrated that the drug, which is developed for the treatment of psoriasis and prepared in the above-mentioned formulation, is effective in psoriasis pathophysiology by testing it on the IMQ-mouse model in a similar manner to the samples in the literature.

The activity of the product can be demonstrated by the graphical representations generated during the development of the invention as follows:

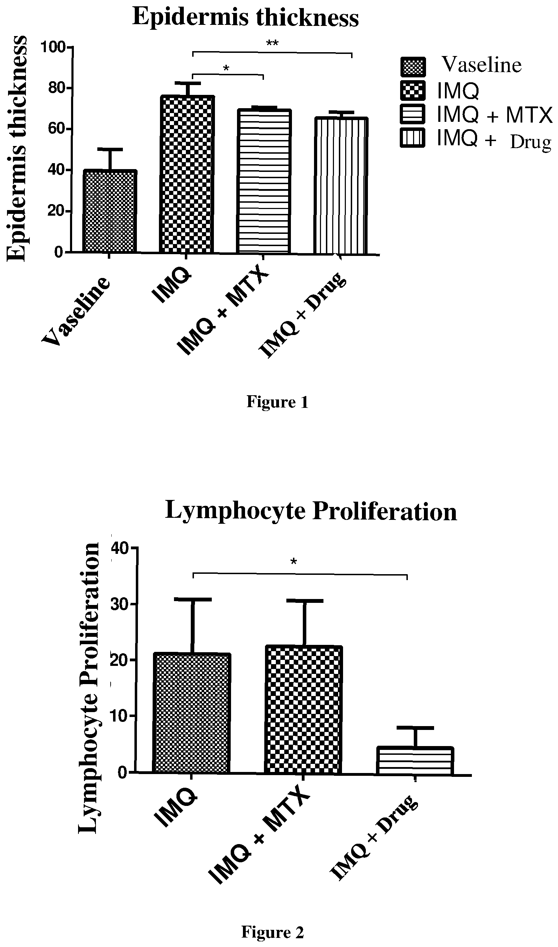

- **Figure 1.**: In the experiments performed on mice, a significant decrease was observed in epidermis thickness compared to the control group as a result of the drug administration in the measurements conducted on samples taken from IMQ-administered skin. It was reported that this decrease was more significant compared to the MTX-administered mice.
- **Figure 2.**: Lymphocyte proliferation is coherent with psoriasis pathology. It was observed that cell proliferation in the drug administered mice significantly decreased compared to the IMQ and MTX-administered mice.

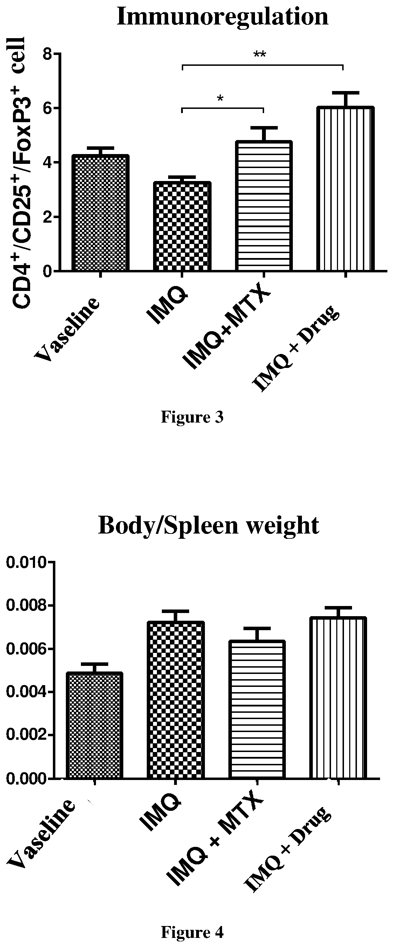

- **Figure 3.**: In the lymphocyte cultures where T regulator cell analysis was performed, it was found that the number of CD4⁺/CD25⁺/FoxP3⁺ cells were higher in the drug-administered mice than the IMQ-administered mice.
- **Figure 4.**: A significant difference was not observed between the groups in terms of the body/spleen weight index.

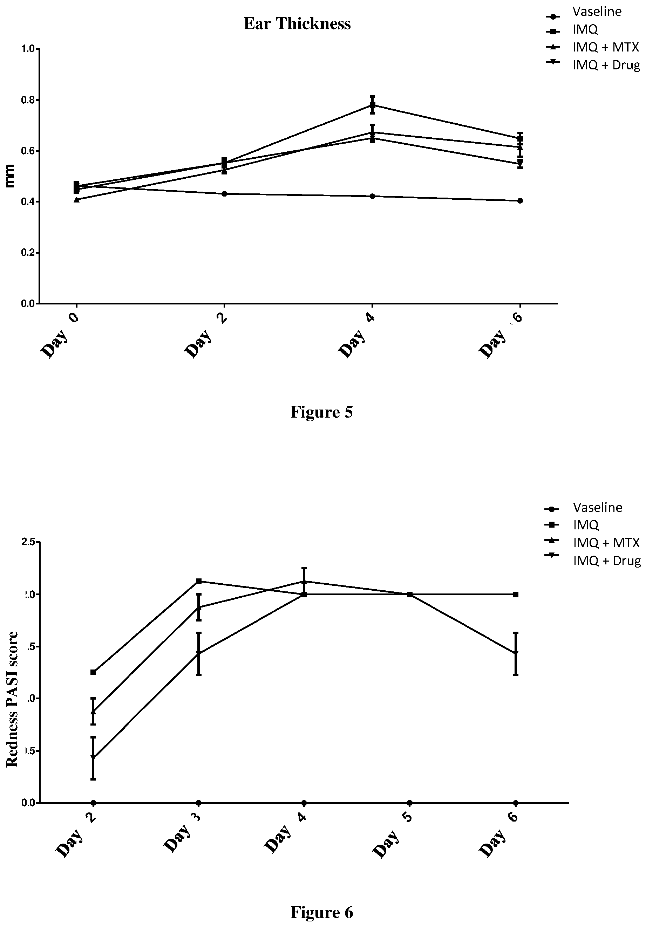

- **Figure 5.**: As a result of the measurements conducted at the IMQ-administered regions in the ears of the mice throughout the experiment, no change was detected in the Vaseline-applied group, whereas thickening occurred in other groups; however no significant difference was observed between the groups.
- **Figure 6.**: According to the redness PASI score evaluation of the IMQ-administered region on the back of the mice, it was observed that the redness decreased in the drug-administered mice as of the 6^{th} day. According to the thickness PASI score evaluation of the IMQ-administered region on the back of the mice, it was determined that the thickness decreased in both MTX (as of the 5^{th} day) and drug group (as of the 4^{th} day).

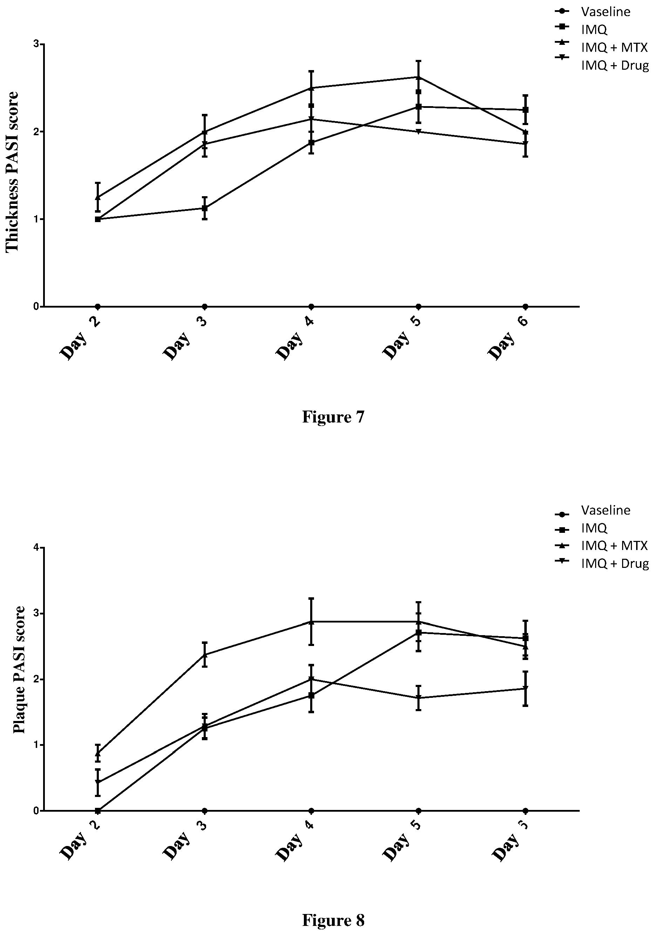

- **Figure 7.**: According to the plaque formation PASI score evaluation of the IMQ-administered region on the back of the mice, it was observed that the scaling decreased in both MTX and drug groups as of the 4^{th} day.
- **Figure 8.**: Decrease was observed in both MTX and drug groups as of the 4^{th} day according to the total PASI score evaluation.

### REFERENCES

**[1].** Walter, A., Schäfer, M., Cecconi, V., Matter, C., Urosevic-Maiwald, M., Belloni, B., Schönewolf, N., Dummer, R., Bloch, W., Werner, S., Beer, H. D., Knuth, A. and van den Broek, M., 2013. Aldara activates TLR7-independent immune defence. Nature Communications, 4, 1560.
**[2].** Nadeem, A., Al-Harbi, N. O., Al-Harbi, M. M., El-Sherbeeny, A. M., Ahmad, S. F., Siddiqui, N., Ansari, M. A., Zoheir, K. M., Attia, S. M., Al-Hosaini, K. A. and Al-Sharary, S. D., 2015. Imiquimod-induced psoriasis-like skin inflammation is suppressed by BET bromodomain inhibitor in mice through RORC/IL-17A pathway modulation. Pharmacological Research, 99, 248-257.
**[3].** Arora, N., Shah, K. and Pandey-Rai, S., 2016. Inhibition of imiquimod-induced psoriasis-like dermatitis in mice by herbal extracts from some Indian medicinal plants. Protoplasma, 253 (2), 503-515.
**[4].** Chen, H. H., Chao, Y. H., Chen, D. Y., Yang, D. H., Chung, T. W., Li, Y. R. and Lin, C. C., 2016. Oral administration of acarbose ameliorates imiquimod-induced psoriasis-like dermatitis in a mouse model. International immunopharmacology, 33, 70-82.
**[5].** Di, T. T., Ruan, Z. T., Zhao, J. X., Wang, Y., Liu, X., Wang, Y. and Li, P., 2016. Astilbin inhibits Th17 cell differentiation and ameliorates imiquimod-induced psoriasis-like skin lesions in BALB/c mice via Jak3/Stat3 signaling pathway. International immunopharmacology, 32, 32-38.
**[6].** Jia, H. Y., Shi, Y., Luo, L. F., Jiang, G., Zhou, Q., Xu, S. Z. and Lei, T. C., 2016. Asymmetric stem-cell division ensures sustained keratinocyte hyperproliferation in psoriatic skin lesions. International journal of molecular medicine, 37(2), 359-368.
**[7].** Krueger, J. G. and Bowcock, A., 2005. Psoriasis Pathophysiology: Current Concepts of Pathogenesis. Ann Rheum Dis, 64, il30.

## Claims

1. A medicine for use in the treatment of psoriasis **characterized in that** it comprises tallow, larch resin, beeswax, gum mastic, propolis, Alkanna tinctoria, alum and Juniper tar.

2. A medicine for use in the treatment of psoriasis according to Claim 1 **characterized in that** it comprises 800 to 1000 g of tallow, 250 to 350 g of larch resin, 450 to 550 g of beeswax, 2 to 3 g of gum mastic, 40 to 50 g of propolis, 200 to 250 g of Alkanna tinctoria, 200 to 250 g of alum, and 150 to 200 g of Juniper tar.

3. A medicine for use in the treatment of psoriasis according to Claim 1 **characterized in that** it is applied as an ointment on the surface of the psoriasis patient's skin where the lesion is observed.

4. A method for producing the medicine for treatment of psoriasis according to any one of the preceding claims, **characterized by** the steps of
- melting the tallow at 300°C by continuously mixing it with the beeswax,
- adding first the gum mastica and then the propolis to the obtained molten mixture,
- adding ground larch resin to the mixture together with alum,
- finally adding ground Alkanna tinctoria and Juniper tar to the mixture,
- obtaining a homogenous mixture by means of continuous mixing,
- filtering the homogenous mixture,
- obtaining the medicine for treatment of psoriasis, which is the final product in the form an elute.

## Patentansprüche

1. Ein Arzneimittel zur Verwendung bei der Behandlung von Psoriasis, **dadurch gekennzeichnet, dass** es Talg, Lärchenharz, Bienenwachs, Mastix, Propolis, Alkanna tinctoria, Alaun und Wacholderteer enthält.

2. Ein Arzneimittel zur Verwendung bei der Behandlung von Psoriasis nach Anspruch 1, **dadurch gekennzeichnet, dass** es 800 bis 1000 g Talg, 250 bis 350 g Lärchenharz, 450 bis 550 g Bienenwachs, 2 bis 3 g Mastix, 40 bis 50 g Propolis, 200 bis 250 g Alkanna tinctoria, 200 bis 250 g Alaun und 150 bis 200 g Wacholderteer enthält.

3. Ein Arzneimittel zur Verwendung bei der Behandlung von Psoriasis nach Anspruch 1, **dadurch gekennzeichnet, dass** es als Salbe auf die Hautoberfläche des Psoriasispatienten an der Stelle aufgetragen wird, an der die Läsion festgestellt wird.

4. Ein Verfahren zur Herstellung des Arzneimittels zur Behandlung von Psoriasis nach einem der vorhergehenden Ansprüche, **gekennzeichnet durch** die folgenden Schritte:
- Schmelzen des Talgs bei 300 °C unter kontinuierlichem Vermischen mit dem Bienenwachs,
- zunächst Zugeben des Mastix und anschließend des Propolis zu der erhaltenen geschmolzenen Mischung,
- Zugeben von gemahlenem Lärchenharz zusammen mit Alaun zu der Mischung,
- schließlich Zugeben von gemahlener Alkanna tinctoria und Wacholderteer zu der Mischung,
- Herstellen einer homogenen Mischung durch kontinuierliches Mischen,
- Filtern der homogenen Mischung,
- Erhalten des Arzneimittels zur Behandlung von Psoriasis als Endprodukt in Form eines Eluats.

## Revendications

1. Médicament destiné à être utilisé dans le traitement du psoriasis, **caractérisé en ce qu'**il comprend du suif, de la résine de mélèze, de la cire d'abeille, de la gomme mastic, de la propolis, de l'*Alkanna tinctoria*, de l'alun et du goudron de genièvre.

2. Médicament destiné à être utilisé dans le traitement du psoriasis selon la revendication 1, **caractérisé en ce qu'**il comprend de 800 à 1000 g de suif, de 250 à 350 g de résine de mélèze, de 450 à 550 g de cire d'abeille, de 2 à 3 g de gomme mastic, de 40 à 50 g de propolis, de 200 à 250 g d'*Alkanna tinctoria*, de 200 à 250 g d'alun et de 150 à 200 g de goudron de genièvre.

3. Médicament destiné à être utilisé dans le traitement du psoriasis selon la revendication 1, **caractérisé en ce qu'**il est appliqué sous forme de pommade sur la surface de la peau du patient atteint de psoriasis où la lésion est observée.

4. Procédé de préparation du médicament destiné au traitement du psoriasis selon l'une quelconque des revendications précédentes, **caractérisé par** les étapes de
- faire fondre le suif à 300°C en le mélangeant continuellement avec la cire d'abeille,
- ajout tout d'abord de la gomme mastic, puis de la propolis au mélange fondu obtenu,
- ajout de la résine de mélèze broyée au mélange avec l'alun,
- ajout enfin de l'*Alkanna tinctoria* broyée et du goudron de genièvre au mélange,
- obtention d'un mélange homogène par mélange continu,
- filtration du mélange homogène,
- obtention du médicament destiné au traitement du psoriasis, constituant le produit final sous forme d'éluat.
